Europäisches Patentamt

European Patent Office

Office européen des brevets

(11) Numéro de publication: **0 236 219**
**A1**

(12) **DEMANDE DE BREVET EUROPEEN**

(21) Numéro de dépôt: **87400397.3**

(22) Date de dépôt: **23.02.87**

(51) Int. Cl.4: **A 61 K 7/155**

(30) Priorité: **24.02.86 FR 8602512**

(43) Date de publication de la demande:
**09.09.87 Bulletin 87/37**

(84) Etats contractants désignés:
**BE CH DE ES GB IT LI LU**

(71) Demandeur: **LABORATOIRE DE COSMETOLOGIE MODERNE ARSENE VALERE en abrégé L.C.M. Arsène Valere**
**rue Barthélémy-Thimonier Z.I. Nord**
**F-87021 Limoges Cédex (FR)**

(72) Inventeur: **Moreau, Pierre**
**23 rue Edmond About**
**F-87000 Limoges (FR)**

(74) Mandataire: **Harlé, Robert**
**c/o Cabinet Harlé & Phélip 21, rue de la Rochefoucauld**
**F-75009 Paris (FR)**

(54) **Cire à épiler réutilisable et son procédé d'obtention.**

(57) Cire à épiler réutilisable et son procédé d'obtention.
L'invention a pour objet une cire à épiler réutillisable comprenant de la colophane et de la cire d'abeille qui contient, en outre, un adjuvant noir à base de charbon ayant pour effet lors de sa réutilisation tant de masquer les poils et leurs odeurs que d'éviter une filtration.

EP 0 236 219 A1

Bundesdruckerei Berlin

## Description

Cire à épiler réutilisable et son procédé d'obtention

La présente invention concerne une cire à épiler réutilisable et son procédé d'obtention.

On sait qu'il existe à l'heure actuelle deux types de cire à épiler, d'une part les cires dites froides et d'autre part les cires dites chaudes. Ces types de cires correspondent en fait à leur mode d'utilisation, dans le premier cas elles sont utilisées à froid, donc sans chauffage alors que dans le second cas elles sont préalablement chauffées avant d'être utilisées et appliquées dans un état pâteux visqueux à température convenable.

La présente invention concerne une cire chaude.

Les cires chaudes se présentent habituellement sous la forme de pains de cire. Lors de l'utilisation, les pains de cire sont introduits dans un dispositif de chauffage. Lorsque la cire se trouve dans un état pâteux, l'utilisatrice applique sur la partie à épiler, à l'aide par exemple d'une spatule une partie du mélange pâteux visqueux qui en refroidissant va constituer une bande. Les poils sont éliminés après l'arrachage des bandes de cire appliquées par l'utilisatrice.

A la fin de l'épilage, les bandes de cire sont réintroduites dans l'appareil de chauffage et forment après refroidissement un bloc de cire dans lequel se trouvent insérés les poils qui ont été arrachés.

Après plusieurs utilisations, quelquefois après une seule utilisation, il est nécessaire de filtrer la cire pour en éliminer les poils. Une telle opération constitue un inconvénient sérieux pour l'utilisatrice.

D'une part il s'agit d'une opération désagréable car les poils présentent souvent une forte odeur, d'autre part l'opération de filtration n'est pas toujours aisée à réaliser car les poils sont incrustés dans le bloc de cire.

La présente invention a pour objet de remédier aux inconvénients précités.

Conformément à la présente invention, il n'est plus nécessaire de filtrer la cire après une ou plusieurs utilisations car la composition de la cire comprend un adjuvant ou additif qui a un effet sur les poils.

La présente invention propose donc une cire à épiler réutilisable comprenant de la colophane et de la cire d'abeille qui est caractérisée en ce qu'elle contient, en outre, un adjuvant noir à base de charbon ayant pour effet lors de sa réutilisation tant masquer les poils et leurs odeurs que d'éviter une filtration.

La présente invention vise également un procédé d'obtention de cire à épiler réutilisable qui consiste à fondre ensemble la colophane et la cire d'abeille, à porter ledit mélange à une température où celui-ci est à l'état liquide et à agiter ledit mélange pour bien l'homogénéiser qui est caractérisé en ce qu'il consiste à incorporer progressivement dans ledit mélange un adjuvant noir à base de charbon tout en maintenant le chauffage et l'agitation jusqu'au dégazage complet du mélange puis à conditionner.

L'invention concerne également les caractéristiques ci-après considérées isolément ou selon toutes leurs combinaisons techniquement possibles :

- l'adjuvant noir à base de charbon est du noir animal purifié;
- l'adjuvant noir à base de charbon est du charbon actif végétal;
- la cire comprend de 70 à 75 parties en poids de colophane, de 20 à 25 parties en poids de cire d'abeille et de 1 à 5 parties en poids d'adjuvant noir à base de charbon;
- l'adjuvant noir à base de charbon a une granulométrie comprise dans la gamme du micron au centième de millimètre.

L'invention sera mieux comprise à l'aide des deux exemples de mise en oeuvre ci-après.

EXEMPLE 1

On fond ensemble de la colophane et de la cire d'abeille dans un rapport pondéral de 3:1. On porte le mélange à une température d'environ 120°C. On agite ledit mélange pour bien l'homogénéiser. On incorpore alors progressivement de 1 à 5% en poids par rapport audit mélange colophane-cire d'abeille de charbon activé officinal (carbo-activus). Il s'agit d'une poudre noire d'une granulométrie comprise entre 1 micron et quelques centièmes de millimètre qui est un charbon végétal. On maintient le chauffage et l'agitation jusqu'au dégazage complet du mélange. Après refroidissement on procède aux opérations habituelles de conditionnement.

La cire obtenue est de couleur noire.

EXEMPLE 2

On répète le mode opératoire de l'exemple 1 si ce n'est qu'on utilise à la place du charbon activé officinal (carbo-activus) du charbon animal purifié (carbo-ossium depuratus). Ce charbon animal purifié résulte du traitement du charbon animal ordinaire pulvérisé avec de l'acide chlorhydrique dilué. Le charbon animal purifié se présente sous la forme d'une poudre noire d'une granulométrie comprise entre 1 micron et quelques centièmes de millimètre.

La cire obtenue est de couleur noire.

La cire selon la présente invention du fait de l'incorporation de l'adjuvant noir à base de charbon n'évolue pas avec les utilisations successives et ne prend pas d'odeur désagréable.

Grâce à l'addition dudit adjuvant noir à base de charbon, il n'est plus nécessaire de procéder à une quelconque filtration lors des nouvelles utilisations de la cire car les poils ne sont plus visibles.

L'invention n'est pas limitée aux modes de réalisation décrits et elle englobe les équivalents techniques qui rentrent dans son cadre.

## Revendications

1. Cire à épiler réutilisable comprenant de la colophane et de la cire d'abeille, caractérisée en ce que'elle contient, en outre, un adjuvant noir à base de charbon ayant pour effet lors de sa réutilisation tant de masquer les poils et leurs odeurs que d'éviter une filtration.

2. Cire à épiler réutilisable selon la revendication I, caractérisée en ce que l'adjuvant noir à base de charbon est du noir animal purifié.

3. Cire à épiler réutilisable selon la revendication I, caractérisée en ce que l'adjuvant noir à base de charbon est du charbon actif végétal.

4. Cire à épiler réutilisable selon l'une quelconque des revendications I à 3, caractérisée en ce qu'elle comprend de 70 à 75 parties en poids de colophane, de 20 à 25 parties en poids de cire d'abeille et de I à 5 parties en poids d'adjuvant noir à base de charbon.

5. Cire à épiler réutilisable selon l'une quelconque des revendications I à 4, caractérisée en ce que l'adjuvant noir à base de charbon a une granulométrie comprise dans la gamme du micron au centième de millimètre.

6. Procédé d'obtention de cire à épiler réutilisable qui consiste à fondre ensemble la colophane et la cire d'abeille, à porter ledit mélange à une température où celui-ci est à l'état liquide et à agiter ledit mélange pour bien l'homogénéiser, characterisé en ce qu'il consiste à incorporer progressivement dans ledit mélange un adjuvant noir à base de charbon tout en maintenant le chauffage et l'agitation jusqu'au dégazage complet du mélange puis à conditionner d'une façon en soi connue.

7. Procédé selon la revendication 6, caractérisé en ce que l'adjuvant noir à base de charbon est du noir animal purifié.

8. Procédé selon la revendication 6, caractérisé en ce que l'adjuvant noir à base de charbon est du charbon actif végétal.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | BE-A- 468 839 (J.A. UYTDENHOEF) * En entier * | 1-8 | A 61 K 7/155 |
| A | CH-A- 176 933 (H. SIBER) * Revendications 1,3 * | 1-8 | |
| A | FR-A-2 137 112 (J. DUPUY) * Page 6, lignes 24-27; revendication 8 * | 1-8 | |

DOMAINES TECHNIQUES RECHERCHES (Int. Cl.4)

A 61 K

Le présent rapport de recherche a été établi pour toutes les revendications

| Lieu de la recherche | Date d'achèvement de la recherche | Examinateur |
|---|---|---|
| LA HAYE | 01-06-1987 | BERTE M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82